# EUROPEAN PATENT SPECIFICATION

(11) **EP 2 720 654 B1**
(45) Date of publication and mention of the grant of the patent: **06.04.2016**
(21) Application number: 12730623.1
(22) Date of filing: 06.04.2012
(51) Int. Cl.: A61F 5/08

(54) **DEVICE TO NARROW THE COLUMELLA AND TO DILATE THE NASAL VALVE**
VORRICHTUNG ZUR VERENGUNG DER COLUMELLA UND ZUM DILATIEREN DER NASENKLAPPE
DISPOSITIF POUR RÉTRÉCIR LA COLUMELLE ET DILATER LA VALVE NASALE

(30) Priority: 07.04.2011 IT BO20110182
(43) Date of publication of application: 23.04.2014
(73) Proprietor: Mezzoli, Giorgio, 48022 Lugo (ra) (IT); Libra, Gaetano, 40024 Castel San Pietro Terme (bo) (IT)
(72) Inventor: Mezzoli, Giorgio, 48022 Lugo (ra) (IT); Libra, Gaetano, 40024 Castel San Pietro Terme (bo) (IT)
(74) Representative: Roncuzzi, Davide
(86) International application number: PCT/IB2012/051723
(87) International publication number: WO 2012/137182

(56) References cited:
- EP-A1- 0 958 798
- WO-A1-2007/119041
- DE-A1- 19 736 717
- US-A- 5 931 852

## Description

### FIELD OF THE INVENTION:

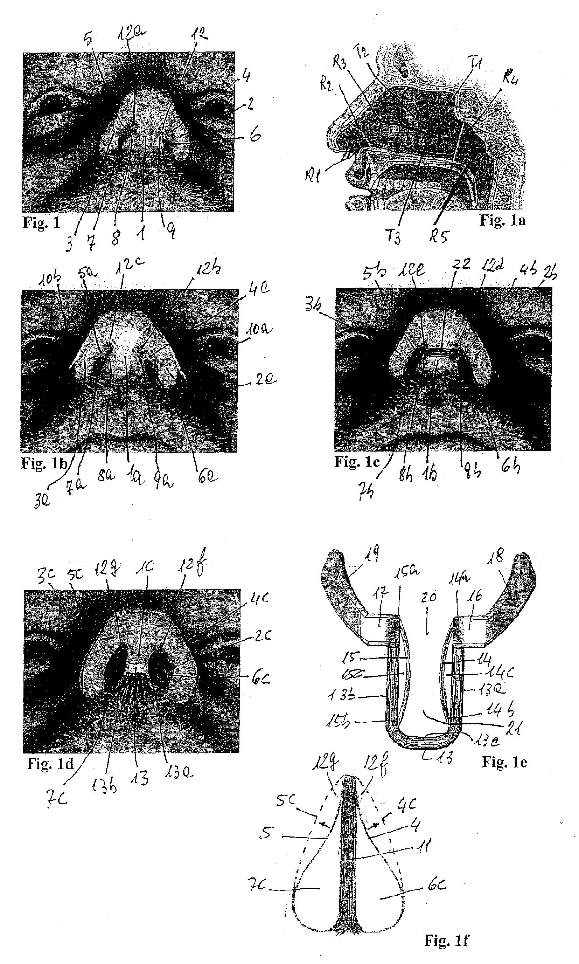

The present invention deals with a device to narrow the columella (1) and, at the same time, to dilate the nasal valve (12) and (12a) in order to decrease the resistance which these two internal structures, located at the beginning of each nasal cavity (Figures 1d and 1f), oppose to the air passage through each nasal cavity in order to improve the respiration through both nasal cavities in normal breathing and particularly in special situations such as during sleep, during sports or during the outpouring of a kiss.

### PRIOR ART:

Henceforth, the term "proximal" means proximal with respect to the user and "distal" distal with respect to the user. Devices to facilitate breathing through the nasal cavities currently on the market consist of devices that dilate only the nasal valve either from the inside of the nasal cavity or from the outside.

### Device dilating the nasal valve from the inside.

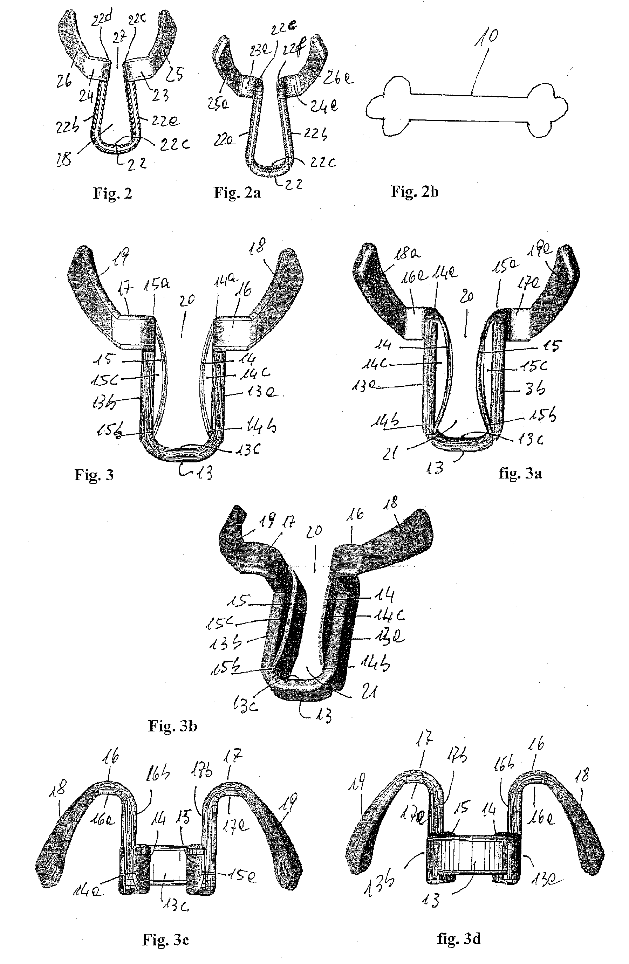

This first version of known device is described in Figures 2, 2a and it consists of two horizontal branches (22a) and (22b), joined by a portion (22) at the distal end and which delimit a space (28) while, at the proximal end (22c) and (22d), the two branches (22a) and (22b) delimit a very small space (27). At the proximal end (22c) and (22d) each of the two -right (22a) and left (22b)- branches has a upwards portion of 5 mm respectively (22e) and (22f) which, at the apex (23) and (24), folds up towards the outside, acute angled (23a) and (24a), on the right (23) and on the left (24), and it continues laterally, in the form of a dragonfly wing, on the right (25) and on the left (26), with supero-lateral convexity. Figure 1c shows the known device inserted into the nasal vestibule and the nasal valve where it should be noted that the two horizontal branches (22a) and (22b) distally joined by a portion (22) are insufficient to narrow the thickness of the columella (1), measuring 3.5 mm high. This first version of device (10) has the drawback that the proximal end (22d) of the left branch (22b) and the proximal end (22c) of the right branch (22a), press on the mucosa of both sides - right and left - of the nasal septum (11), being very close to each other; it causes lesions either during the insertion, due to the friction on the septum mucosa, either because it causes ischemic and / or sores mucosal injury of the septum mucosa below the proximal end (22c) of the right branch (22a) and the proximal end (22d) of the left branch (22b), once positioned in the operating conditions, exerting pressure on the mucosa directly without interposition of soft material. Moreover, at the distal end, the device has no manual grip, to dilate the two horizontal branches (22a) and (22b) and to improve the manual insertion around the columella (1) and the septum (11) so that the operation turns out to be difficult and painful.

### Device dilating the nasal valve from the outside:

Figure 1b shows this second version of known device (10 ', 10a), described in Figure 2b, whose internal wall is applied on the skin, provided with an adhesive substance, above the vestibule and the nasal valve and, as it is constructed with plastic and elastic material, it tends to return to horizontal position, by dragging and expanding outward the wings of the nose - right (3a) and left (2a) -, and the side walls of the nasal valve - right (5a) and left (4a), dilating slightly even the nasal valve - right (12c) and left (12b). This second version of device (10'), (10a) presents the drawback that it can become detached from the back of the nose if it is wet, as in the case of abundant sweating during sports, or during sleep, turning on the pillow and rubbing it repeatedly on the pillow. Furthermore this second version of device (10'), (10a) does not narrow the columella (1), hence in all cases where there is a very large columella, as in Figure 1, the air passage through the right (7a) and left (6a) nasal vestibule is limited. Even the dilation of lateral wall (4a) and (5a) of the nasal valve (12c) and (12b) turns out to be not very effective as shown in Figure 1 b. A purpose of the present invention is to obviate the above mentioned drawbacks of the known devices to dilate the nasal valve.

### SUMMARY OF THE INVENTION

According to the present invention, this purpose is achieved with a device having the features according to claim 1. Further characteristics of the device are subject of the dependent claims.

### LIST OF FIGURES:

The invention will be now better described with reference to some non-limiting embodiments shown in the attached drawings, wherein:
Figure 1 shows a front view and a bottom view of the nasal distal end.
Figure 1 a shows a view of the lateral wall of the right nasal cavity.
Figure 1b shows the application of the known device (10) described in
Figure 2b, which dilates the nasal valve from the outside, on the skin of the distal portion of the nasal pyramid, at the level of the nasal wings (2a) and (3a),.
Figure 1c shows the application, within the vestibule and the nasal valve, of the known device described in Figures 2 and 2a, which dilates the nasal valve from the inside.
Figure 1d shows an example of application of a first embodiment of a device according to the invention, within the vestibule and the nasal valve.
The Figure 1e shows a top view of the device described in Figure 1d.
Figure 1f shows a schematic image of the nasal valve dilation.
Figures 2, 2a show top and bottom views of the known device described in
Figure 1c, which dilates the nasal valve from the inside.
Figure 2b shows a top view of the known device (10) described in Figure 1b which dilates the nasal valve from the outside.
Figures 3, 3a, 3b, 3c and 3d show top, bottom, latero-distal from the top, frontal views of the proximal and distal ends described in Figure 1e;

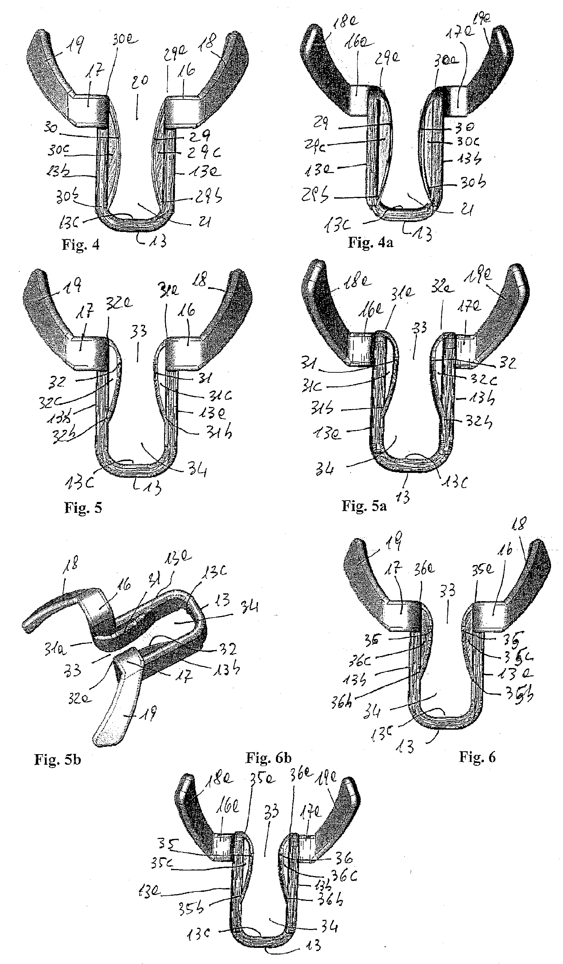

Figures 4, 4a show top and bottom views of a variant of the device described in Figures 3 through 3d, according to a second embodiment of the invention.
Figures 5, 5a, 5b show top, bottom and latero-proximal views of a third embodiment according to the invention.
Figures 6, 6b show top, bottom views of a variant of the device described in Figure 5 and 5a, according to a fourth embodiment of the invention.

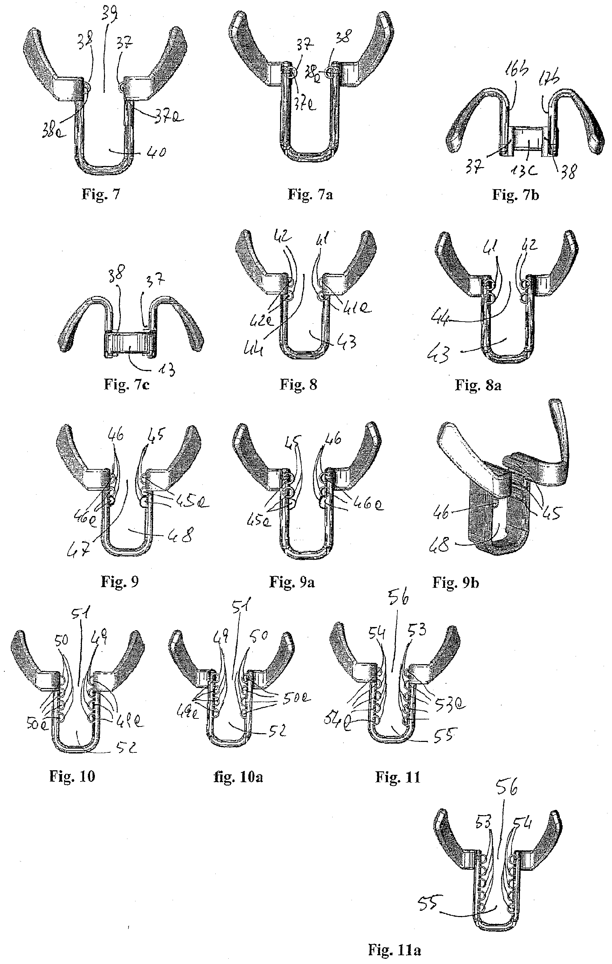

Figures 7, 7a, 7b and 7c show top, bottom, frontal views of the proximal and distal ends, of a fifth embodiment of a device according to the invention.
Figures 8, 8a, show top, bottom views of a version different to the previous device, according to a sixth embodiment of the invention.
Figures 9, 9a 9b show top and bottom views of a further version of the device different to any previous one, according to a seventh embodiment of the invention.
Figures 10, 10a, show a top, bottom and top-lateral views of another version of the device different to any previous one according to an eighth embodiment of the invention.
Figures 11 and 11 a show respectively a top and a bottom view of a version, different to any previous one according to a ninth embodiment of the invention.

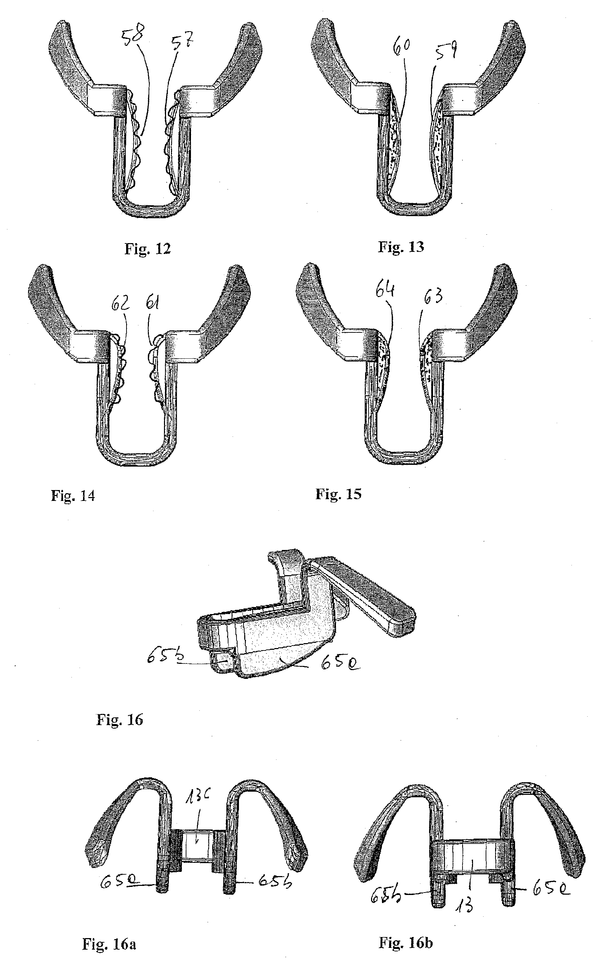

Figure 12 shows a top view of a version of the device described in Figures 3 through 3d, 4 and 4a, according to a tenth embodiment of the invention.
Figure 13 shows a top view of a version of the device described in Figures 3 through 3d, 4 and 4a, according to an eleventh embodiment of the invention.
Figure 14 shows a top view of a version of the device described in Figures 5 through 5b, 6 and 6a, according to a twelfth form of the invention.
Figure 15 shows a top view of another version of the device described in Figures 5 through 5b, 6 and 6a, according to a thirteenth form of the invention.

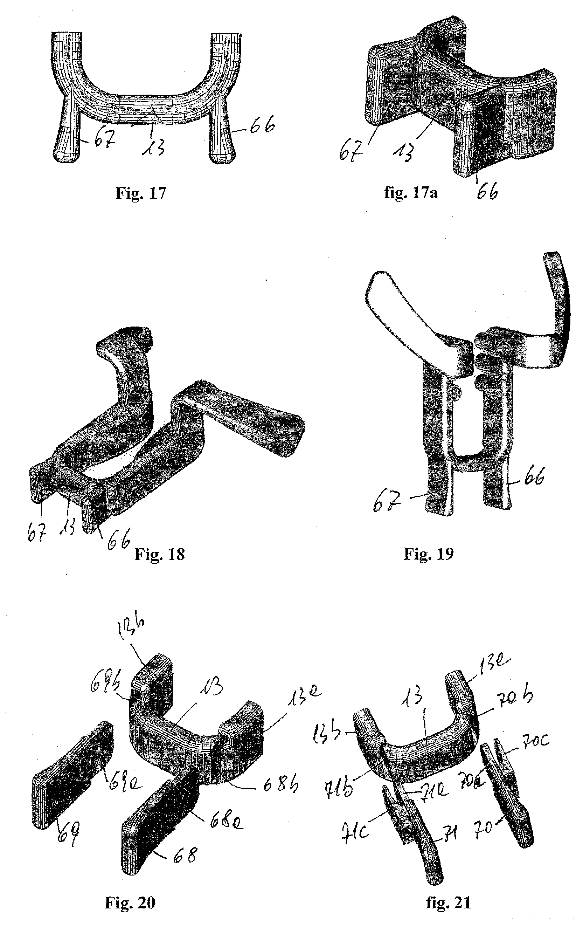

Figures 16 through 16b show lateral, proximal and distal frontal views, of a further version of the devices described above, according to a fourteenth embodiment of the invention. Figures 17 and 17a show top and lateral-distal views of a version of the distal portion different in any previous device, according to a fifteenth embodiment of the invention.
Figure 18 shows a top-lateral view of the version described in Figures 17 and 17a according to a sixteenth embodiment of the invention, applicable to all the devices described above.
Figure 19 shows a top-lateral view of the version described in Figures 17 and 17a applied to the devices described in Figures 7 through 7c, 8 and 8a, 9 through 9b, 10 and 10a, 11 and 11a, respectively according to a seventeenth, eighteenth, nineteenth, twentieth and twenty-first embodiment of the invention.
Figure 20 shows a top view of a version described in Figures 17 and 17a, according to a twenty-second embodiment of the invention.
Figure 21 shows a top view of a version described in Figure 20 according to a twenty-third embodiment of the invention.

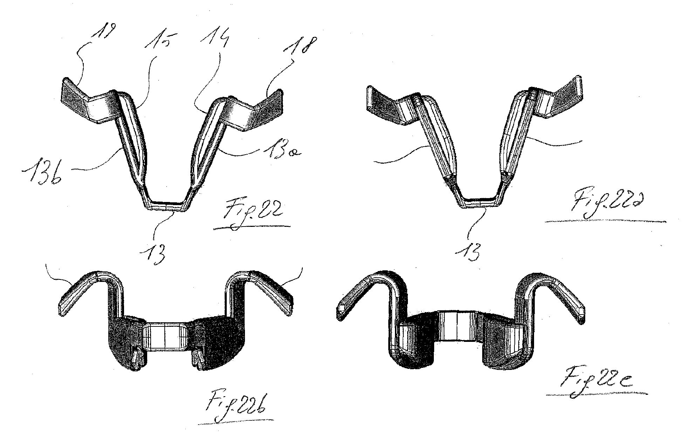

Figures 22 through 22c show top, bottom, frontal views of the distal end and the proximal end of a version of the device described in Figures 3 through 3d according to a twenty-fourth embodiment of the invention.

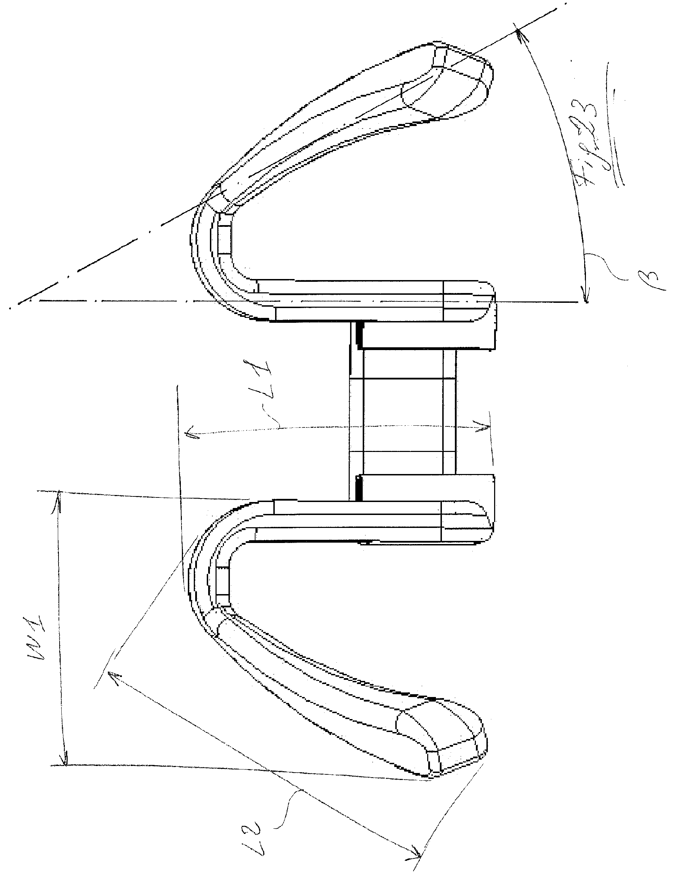

Figure 23 shows a frontal view of the device described in Figure 3;

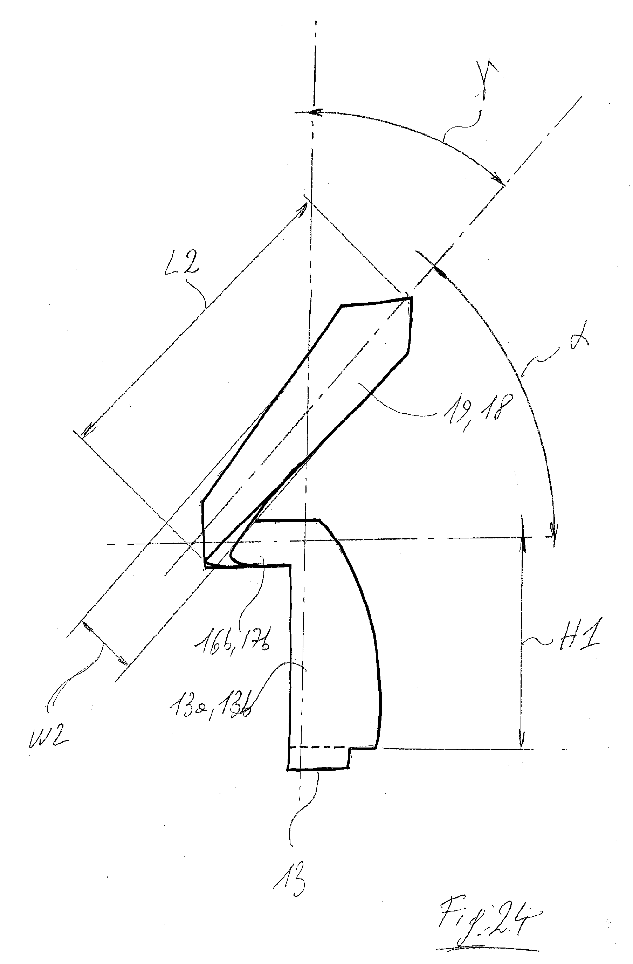

The Figure 24 shows a lateral view of the device described in Figure 3.

### DETAILED DESCRIPTION OF THE INVENTION

The nasal vestibule, shown in Figures 1, 1a, 1b, 1c and 1d is the external part of the nasal airways; the anatomical structures that delimit the nasal vestibule are constituted medially by the lateral wall of the columella (1), to the right (8) and to the left (9), and by the mucosa of the nasal septum (11) schematically represented in Figure 1f, and laterally by the right (3) and the left (2) nasal wing. These anatomical structures give funnel shape to the nasal vestibule, with elliptical or oval cross-section, whose coronal or cross sectional area shrinks from the outside towards the inside in each nasal cavity; it has the function of conveying the inspiratory air inside each nasal cavity; the anatomical conformation of the nasal vestibule is the first point of nasal resistance (R1) to the air flow through each nasal cavity, influencing mainly the inspiratory flow rate and secondly the exhalation flow rate. The valve area or internal orifice (R2) shown in Figure 1 a is the crossing point between the nasal vestibule (R1) and the nasal cavity (R3). The distal portion of the valve schematically also represented in Figure 1f is called right (12a) and left (12) nasal valve and it is bounded on the right and on left by the junction between the rigid component, the distal portion of the nasal septum (11), schematically shown in Figure 1f, medially, and the elastic component, constituted by the right (5) and left (4) latero-proximal edge of the nasal wing, laterally; the anatomical structure regulates the direction and the rate of the air that flows through each nasal cavity during the inspiratory and the expiratory phase; the nasal valve (12) and (12a) represents the point of greater resistance (R2) to the air flow during the inspiratory phase, mainly (70% of the total nasal resistance (Rnt) through nasal cavity and 44% of the total respiratory resistance). Any anatomical alteration which results in a decrease of the useful space for air passage (7) and (6) through the nasal vestibule, for example due to an abnormally large columella or a deformed columella due to an alteration of the distal portion of the nasal septum, or through the nasal valve, for example due to a nasal valve collapsed on the septum nasal, or due to both of them at the same time, causes an increase in Nasal resistance (Rn) and therefore greater difficulty in nasal breathing with symptoms manifestation such as snoring during sleep, increased of respiratory distress getting worse during physical exertion, during the outpouring of a kiss or in any other situation where an increased nasal breathing capacity is required.

Figure 1 shows a bottom view of the nasal vestibule. A large columella (1) limits the space for the air passage through the nasal vestibule both to the right (7) and to the left (6). In fact, the right lateral edge (8) of the columella (1) and the right wing of the nose (3) delimit a very small space (7) as well as the left lateral edge (9) of the columella (1) and the left wing of the nose (2) delimit a small space (6). The collapse toward the nasal septum (11) of the elastic and the dynamic component of the nasal valve constituted by the lateral-proximal edge of the right (5) and left (4) lateral branch of the nasal wing determines a further reduction of the useful space for the air passage both to the right (7) and to the left (6).

Figure 1a shows a view of the lateral wall of the right nasal cavity where the anatomical structures are present, in anteroposterior series, and, in each nasal cavity, they delimit the space through which the inspiratory and expiratory air flows and they constitute points of Nasal resistance (Rn) to the air flow: the nasal vestibule (R1), the valve area (R2) whose upper portion is called "nasal valve", the nasal cavity (R3) where upper (T1), medium (T2) and lower (T3) turbinates are present, and the choanal area (R4). The choanal area continues, posteriorly, with the rhynopharyngeal area (R5) where the adenoid tissue and the right and left orifice of the eustachian tube are present which connects the rhynopharynx with the middle ear.
Figure 1d shows a bottom view of a device according to a first embodiment of the invention, described in Figure 1 and in Figures 3 through 3d, inserted into the vestibule and the nasal valve, which determines the narrowing of the columella (1), the separation of the right (3c) and left (2c) nasal wings, and of the latero-proximal edge of the right (5c) and left (4c) nasal wing from the columella (1) and from the nasal septum (11) resulting in the expansion of the right (12g) and the left (12f) nasal valve with the consequent increase of useful space for the air flow through the vestibule and the valve of each right (7c) and left (6c) nasal cavity.

Figures 1d and 1e show a top view of a preferred embodiment of the device according to the invention described in Figures 3 through 3d. Figure 1f shows a schematic picture about how dilation of the nasal valve with the devices described according to the invention occurs, to the right (12g) and to the left (12f) so as to increase the useful space for the air flow through the nasal valve to the right (7c) and to the left (6c).

The Figures 3 through 3d show respectively top, bottom and top-lateral, proximal and distal frontal views of a first embodiment of a device according to the invention including two - right (13th) and left (13b) - horizontal branches, joined by a connecting portion (13) at the distal end, to form preferably a "U". Two lamellae, on the right (14) and on the left (15), reach out from the proximal end (14a) and (15a) of the internal wall of each of the two horizontal branches, respectively, (13a) and (13b), which are joined, at the distal end (14b) and (15b), the internal wall of each of the two horizontal branches, respectively (13a) and (13b), delimiting, medially, a space, open above and below, elongated-leaf shaped: on the right (14c) it is delimited by the internal wall of the branch (13a) laterally, on the left (15c) it is delimited by the wall inside of the branch (13b) laterally. Right (14) and left (15) lamellae in each of the two internal walls of the right (13a) and left (13b) horizontal branches, act as a soft cushion during both the insertion and the extraction step. Moreover, once inserted and located inside the vestibule and the nasal septum, it fits the columella (1) and the nasal septum (11), adhering to the mucosa without altering it, damaging it or causing pain. The external wall of the two lamellae (14) and (15) and the internal wall (13c) of the section (13) that joins, distally, the right branch (13a) to the left branch (13b), delimit a pot-shaped space with a distal base (21) and a proximal neck (20) into which, respectively, the columella (1) and the nasal septum (11) is positioned. A vertical branch departs from the proximal portion of each branch (13a) and (13b), respectively, (16b) and (17b), and it measures 6 mm in height (the height can vary from 4 to 10 mm depending on the dimensions of the vestibule and / or the nasal valve), then it folds, acute angled, towards the outside, respectively (16) and (17), preferably dome-shaped, concave toward the bottom and convex toward the top, then it continues with a dragonfly wing-shaped lamella to the right (18) and (19) to the left. Preferably, second branches 16b, 17b are transverse with the respective first horizontal branches 13a, 13b and, very preferably, they are substantially perpendicular to the latter. The dragonfly wing-shaped lamellae (18), (19) are designed to expand the wings of the nose with respect to the nasal septum, pushing them laterally.

Figure 3c shows a frontal view of the proximal end of the device, according to the invention, which highlights the proximal end (14a) of the lamella (14) which joins the proximal end of the branch (13a) and the proximal end (15a) of the lamella (15) which joins the proximal end of the branch (13b). There are two branches (16b) and (17b) which, after a vertical section preferably of 6 mm. (the height measure can vary from 4 to 10 mm preferably, depending on the size of the vestibule and / or valve nasal) bend outwards acute angled, convex toward the top (16) and (17) and concave toward the bottom (16a) and (17a) and it continues towards the outside (18) and (19). Its form resembles a dragonfly wing. The substantially dragonfly-wing shaped branches (18, 19) have advantageously the same shape of the lateral-superior portion of the alar cartilages that form the exterrnal wall of the nasal valve.

Figure 3d shows a frontal view of the distal end of the device according to the invention which highlights the portion (13) linking, distally, the right horizontal branch (13a) with the left one (13b). Its height measure is preferably 2 or 3 mm., so as to be faint, thus far less than the height of the horizontal branches (13a) and (13b) which measure in height 6 mm preferably. (The measure varies preferably from 4 to 10 mm, as it is a capable measure to narrow the columella (1).
Figures 4 and 4a show respectively top and bottom views of the device according to the invention which differs from that described in Figures 3 through 3d as the lamellae (29) and (30) delimit, respectively, both medially and on the top, a space (29c) and (30c), elongated leaf-shaped, fitting in with the top edge of the right (13a) and left (13b) branch thus setting a pocket - closed at the top and open at the bottom -, in other words the spaces 29c, 30c are closed on the upper side of the device and open on the bottom side of the device.

Figures 5, 5a and 5b show respectively top, bottom and latero-proximal views of a device according to the invention, which differs from that described in Figures 3 through 3d in that the lamellae (31) and (32), which delimit, medially, the elongated-leaf shaped space respectively (31c) and (32c) fit in with the middle portion (31 b) and (32b) of the internal wall of the right (13a) and left (13b) horizontal branch so that the space (34) is wider than that described in Figures 3 through 3d while the space (33) remains almost unchanged.
The wider space (34) is particularly suitable for a very wide columella, possibly
associated with a deviation or anterior displacement of the nasal septum. Figures 6 and 6b show respectively top and bottom view of a device according to the invention which differs from that described in Figures 5, 5a and 5b in that the lamellae (35) and (36) delimit the the elongated-leaf shaped space (35c) and (36c), both medially and on the top, fitting along the top edge of the right (13a) and left (13b) branch and setting, a pocket closed at the top and open at the bottom.

Figures 7 through 7c respectively show top, bottom, proximal and distal frontal views of a device according to the invention which differs from the embodiments above described as, on the internal wall of the proximal end of each of the two - horizontal and parallel to each other - branches (13a) and (13b), there is a lamella (37) and (38) which delimits a vertical empty space (37a) and (38a), half tube-shaped, with medial convexity, consisting of soft, elastic material, such as foamed material; alternatively the lamellae (37), (38) can be made soft and elastic by reducing them appropriately, even if realized with a substantially compact and non-foamed material, for example PVC. The lamellae (37), (38) are suitable for adhering to the mucous membrane of either one of the wall of the nasal septum to prevent the device to be ejected outside.
The expression "half tube-shaped" means that the lamellae (37), (38) have substantially a tubular shape, preferably with a circular, semicircular or otherwise rounded and rotund section.
Figures 8 and 8a respectively show top and bottom view of a device according to the invention which differs from that described in Figures 7 through 7c since there are two lamellae (41) and (42), , on the internal wall of the proximal end of each of the two horizontal branches (13a) and (13b), each of which delimits a vertical empty space (41a) and (42a), half tube-shaped, with medial convexity.

Figures 9, 9a and 9b respectively show top, bottom and latero-proximal from the top views, of a device made according the invention which differs from that described in Figures 8 and 8a since there are three lamellae (45) and (46) on the internal wall of the proximal end of each of the two horizontal branches (13a) and (13b), each of which delimits a vertical empty space (45a) and (46a), half tube-shaped, with medial convexity.
Figures 10 and 10a respectively show top and bottom views of a device according to the invention which differs from that described in Figures 9, 9a and 9b since there are four lamellae (49) and (50) on the internal wall of the proximal end of each of the two horizontal branches (13a) and (13b), each of which delimits a vertical empty space (49a) and (50a), half tube-shaped, with medial convexity.
Figures 11 and 11 a respectively show top and bottom views of a device according to the invention which differs from that described in Figures 10 and 10a since there are five lamellae (53) and (54) on the internal wall of the proximal end of each of the two horizontal and parallel to each other branches (13a) and (13b), each of which delimits a vertical empty space (53a) and (54a), half tube-shaped, with medial convexity.

Figure 12 shows a top view of an embodiment variant of the device according to the invention, which differs from the devices described in Figures 3 through 3d, 4 and 4a, since the lamellae (57) and (58) have a corrugated surface, in order to avoid sores injuries of the nasal mucosa.
Figure 13 shows a top view of an embodiment variant of the device according to the invention, which differs from the devices described in Figures 3 through 3d, 4 and 4a, since the lamellae (59) and (60) are made up of of a spongy substance, in order to prevent sores injury of the nasal mucosa.
Figure 14 shows a top view of an embodiment variant of the device according to the invention, which differs from the devices described in Figures 5 through 5b, 6 and 6b, since the lamellae (61) and (62) present a wavy surface in order to avoid sores injury of the nasal mucosa.
Figure 15 shows a top view of an embodiment variant of the device according to the invention, which differs from the devices described in Figures 5 through 5b, 6 and 6b, since the lamellae (63) and (64) are made up of a spongy substance in order to avoid sores injuries of the nasal mucosa.

Figures 16, 16a and 16b show lateral, frontal, distal-proximal views of a device according to the invention where, at the bottom, each of the two lateral branches (13a) and (13b) is provided with an extension, to the right (65a) and to the left (65b), suitable for narrowing any type of columella (1) more effectively. Such extension is larger in the distal portion, remains unchanged until the median portion and progressively decreases until the proximal portion.
Figures 17 and 17a respectively show top and lateral views of an embodiment variant of the distal end applicable to all devices described above, where, at the distal end, the two horizontal branches (13a, 13b) continue for a few millimeters beyond the portion (13), that join them, setting at least two protrusions or lamellae (66) and (67) whose length measure is preferably 5 mm. (The measure may preferably range from 1 to 10 mm.). With regard to another embodiment, there is a protrusion or central lamina, arranged to extract the nasal dilator as well.
Figure 18 shows lateral, top views of the embodiment of the device according to the invention described in Figures 5, 5a and 5b, 6 and 6b where, as a pure non-limiting example, therefore applicable to all the embodiments of the devices above described, the two horizontal branches (13a) and (13b) are provided, distally, with two lamellae (66) and (67) described in Figures 17 and 17a.

Figure 19 shows a lateral from the top view of the embodiment of the device according to the invention described in Figures 9 through 9b where as a pure non-limiting example, therefore applicable to all the embodiments of the devices previously described in Figures 7 through 7c, 8, and 8a, 10 and 10a, 11 and 11a, the two horizontal branches (13a, 13b) are provided distally with at least two lamellae (66) and (67)
Figure 20 shows a view of the distal end of an embodiment variant of the device according to the invention, which differs from that described in Figures 17 and 17a since the distal end of the two horizontal branches (13a, 13b) are equipped with two moving lamellae (68) and (69) whose proximal portion (68a) and (69a) has to be inserted inside the branches (13a, 13b), when in use.
Figure 21 shows a view of the distal end of an embodiment variant of the device according to the invention, which differs from that described in Figure 20 since each lamella (70) and (71) has a lateral branch (70c) and (71c) respectively, in the proximal portion (70a) and (71a). The proximal end (70a) of the lamella (70) is inserted (70b) within the branch (13a) while the lateral branch (70c) is inserted on its outside, the proximal end (71a) of the lamella (71) is inserted (71b) within the branch (13b) while the lateral branch (71c) is inserted on its outside.

Figures 22 through 22c show respectively top, bottom, distal and proximal frontal views of a device according to the invention which differs from the embodiments described in Figures 3 through 3d since the two branches (13a) and (13b) are divergent from each other, each setting an obtuse angle with the branch (13), and the two branches (13a) and (13b) and the two dragonfly-wings shaped lamellae have a length that ranges between 4 and 15 mm preferably, for example between 4 and 8 mm.
In general, thus regardless of the embodiment described in Figures 22 - 22c, the right (16b) branch and the left branch (17b) have a length L1 preferably between 9 mm and 13 mm, and more preferably between 11 mm and 12 mm (Figure 23).
In general, each second dragonfly-wing shaped lamella (18), (19) has a length L2 preferably between 7 mm and 18 mm, more preferably between 8 mm and 17 mm and, even more preferably between 7.5 mm and 10 mm (Figures 23, 24); longer lamellae penetrate deeper into the nasal valves and they better open them; shorter lamellae are easier and less annoying to insert into the nasal cavities.

In general, each second dragonfly-wing shaped lamella (18), (19) has a preferably variable width W2, along its length, between 3 mm and 7 mm, and more preferably between 4 mm and 5 mm.
In general each of the first two horizontal branches (13a, 13b) has a preferably height H1 between 10 mm and 18 mm, more preferably between 12 mm and 17 mm, and even more preferably between 13 mm and 16 mm. In general each of the cluster formed respectively from A) the second right branch (16b) and from the respective second dragonfly-wing shaped lamella (18), and B) from the second left branch and (17b) from the respective second dragonfly-wing lamella (19), have a width W1 preferably between 8 mm and 11 mm, and more preferably between 9 mm and 10 mm, depending from the width of the device itself.

In general, each second dragonfly-wing shaped lamella (18), (19) lies in an ideal surface having a first inclination α compared to second branches (16b), (17b), preferably between 30° and 60°, more preferably between about 40° and about 50°, and even more preferably approximately equal to 45 ° (Figures 3-24).
Alternatively, in general each second dragonfly-wing shaped lamella (18), (19) lies in an ideal surface having a second inclination γ compared to the first branches (13a), (13b), preferably between 30° and 60°, more preferably between about 40 ° and about 50 ° and, even more preferably approximately equal to 45 °.

In general, each second dragonfly-wing shaped lamella (18), (19) has an inclination β, compared to the relative second branch (16b), (17b) and preferably between 30 ° and 60 °, most preferably between about 40° and about 50°, and even more preferably approximately equal to 45° (Figures 3-23), in an ideal horizontal surface during normal use, when the device is inserted into the user's nose.

Said size ranges and inclinations contribute to make the dilator device according to the invention more comfortable and pleasant to be inserted and held into the nose, and much more effective in opening the nasal valves compared with known devices, for example with respect to those described in documents US5931852 and EP958798A1.
In fact in these prior art documents, the elastic extensions respectively 13, 13 'and 1 would seem to lie in substantially normal surfaces compared to U arms (respectively 14, 14 '; 3).
Instead, lying in inclined surfaces with respect to the first 13a, 13b or to the second branches 16b, 17b, the second dragonfly-wings shaped lamellae (18), (19) of a dilator according to the invention, from the one part allow less flow obstruction of the incoming air in the nostrils, from the other part they have a greater extension along the axis of nasal passages, they penetrate more deeply into the nasal valves, opening them more effectively.

The functioning of the previous devices according to the invention is now described. Under operating conditions Figure 1d, also schematically represented in Figure 1f, the dilators described in Figures 3-21 are positioned within the nasal vestibule up to the valve area or internal ostium. As in the embodiments described in Figures 3-21, the device according to the invention can be molded or otherwise originally produced with the proximal ends 14a, 15a tighter with respect to the thickness of the nasal septum and in such a way that, in undeformed configuration, the first branches 13a, 13b, get next to each another, moving from the connecting portion 13 towards their proximal ends 14a, 15a.
By widening the two right (13a) and left (13b) branches, with the fingers of one hand, by inserting the end (14a) of the right branch (13a) along the left edge (9) of the columella (1) and on the mucosa of the nasal septum left wall (11) and the end (15a) of the left branch (13b) along the right edge (8) of the columella (1) and on the mucosa of the nasal septum right wall (11) in depth into the nasal vestibule, till inserting both the right wing (18) inside the left nasal valve (12f), between the nasal septum (11), medially, and the wing of the nose (2c) and (4c) laterally, and the left wing (19) inside the right nasal valve (12g), between the septum (11), medially, and the wing of the nose (3c) and (5c), laterally, so that the internal wall (13c) of the section (13), which distally joins the two horizontal - right (13a) and left (13b)-branches, clamps the nasal septum and adheres to the external skin of the columella (1), thanks to the elastic return effect of the device material. Therefore the two horizontal branches (13a) and (13b) are inserted within the nasal vestibule up to the valve area or internal ostium, one to the right (13b) and the other to the left (13a) of the nasal septum and of the columella (1), while the two dragonfly wings shaped wings (18) and (19), are positioned on the internal wall of the nasal wings (2) and (3), and then of the nasal valve (12g) and (12f) whereof the nasal wings constitute the supero-lateral wall, separating the wings (2c) and (3c) from the septum (11), dilating the nasal valve (12g) and (12f) so as to significantly reduce the value of the nasal resistance (Rn). In this way, a larger amount of air passes through the vestibule (R1) and the nasal valve (R2) during the nasal breathing, in particular during the inspiratory phase. In a second embodiment of the device according to the invention, under operating conditions, on the distal end, the lamellae (66) and (67) are crushed, the one toward the other, with the fingers of one hand; the two lamellae (66) and (67) spread apart the two horizontal branches (13a) and (13b), behaving substantially as two levers; in that way, the two branches are inserted at the base with greater ease even in presence of a large columella (1) and the device is positioned within the nostrils, introducing the right branch (13a) to the left and the left branch (13b) to the right of the frontal portion of the nasal septum, pushing the two branches within each nasal cavity till the internal part (13e) of the branch (13), which combines, distally, the two horizontal branches (13a) and (13b), is positioned close to the external part of the columella (1). In this way the two horizontal branches (13a) and (13b) embrace the columella (1), from the top to the bottom, narrowing it in its whole height, basis included; meantime, the two wings (18) and (19) of the proximal ends of each horizontal branch (13a) and (13b), separate supero-laterally the nasal wings (2c) and (3c) and the elastic component of the nasal valve represented by the latero-proximal edge (4c) and (5c). In conclusion, the devices above described, on the one hand by narrowing the columella (1) and on the other hand by separating the nasal wings (2c) and (3c) and the elastic component (4c) and (5c) of the valve from the nasal septum (11) get the result of increasing the useful space for the air passage through the vestibule and through the nasal valve (6c) and (7c). All the devices above described are made of soft material such as PVC (no dop), silicone or other soft, but compact and solid material and, at the same time, pliable and flexible. Such result is documented in Figure 1d.

The functioning of the device shown in Figures 22 to 22c is different, as the device according to the invention is molded or however, originally produced in such a way that, in undeformed configuration, the first branches (13a), (13b) diverge and they separate from each other, moving from the linking portion (13) towards their proximal ends (14a), (15a). The device is positioned within the nasal vestibule up to the valve area or internal orifice, moving closer, one towards the other, with the fingers of one hand, the two right (13a) and left (13b) branches, inserting the end (14a) of the right branch (13a) along the left edge (9) of the columella (1) and on the mucosa of the left wall of the nasal septum (11) and the end (15a) of the left branch (13b) along the right edge (8) of the columella (1) and on the mucosa of the right wall of the nasal septum (11) in depth into the nasal vestibule, up to insert both the right wing (18) within the left nasal valve (12f), between the nasal septum (11), medially, and the nasal wing (2c) and (4c) laterally, and the left wing (19) within the right nasal valve (12g), between the septum (11), medially, and the wing of the nose (3c) and (5c), laterally, so that the internal wall (13c) of the portion (13), which joins distally the two - right (13a) and left (13b) - horizontal branches, adheres to the external skin of the columella (1).

In the embodiment described in Figures 22-22c, the branches 13a and 13b are divergent, therefore easier to enter the nasal vestibule, they allow a lower compression of the mucosa of the nasal septum and a best extension of the nasal valve by pushing the nasal wings laterally, from the inside. The nasal valves are held more open, compared to versions described in Figures 3-21 because the horizontal branches 13a, 13b tend to open due to the elastic return of the material. To maintain the dilator stably inserted into the nose, the first lamellae 14, 15 can be advantageously more protruding towards each other and towards the nasal septum; at the same time the first lamellae 14, 15 are suitably pressed against the nasal septum because the wall of each nasal valve tends to push the first horizontal branches against the nasal septum.

The embodiments previously described may be subject to different modifications and variations without departing from the protection field of the present invention.

## Claims

1. Device to narrow the nasal columella (1) and expand the nasal wings (3c) and (2c) and the nasal valve (12g) and (12f), which comprises at least two first horizontal branches (13a) and (13b) joined, at the distal end (13), to form a U; wherein from the internal wall of the proximal end (14a) and (15a), first two - right (14) and left (15) - lamellae branch off, so as to join the internal wall of the respective horizontal branches (13a, 13b) delimiting, medially, a space, to the right (14c) and to the left (15c), respectively, and from the upper edge of the proximal portion of each horizontal branch (13a) and (13b), to the right and to the left, a respective second branch (16b, 17b) gets off; the latter acute angled bends (16, 17), after a short vertical portion, and towards the outside and it continues with a respective second dragonfly-wing shaped lamella (18, 19), to the right and to the left, suitable for being inserted into the nasal vestibule and the nasal valve.

2. Device according to claim 1, **characterized in that** the second dragonfly-wing shaped lamellae, to the right (18) and to the left (19) are suitable for being inserted into the vestibule and the nasal valve so as to dilate the nasal wings, pushing them laterally with respect to the nasal septum.

3. Device according to claim 1, **characterized in that** the right (14c) and left (15c) space delimited by the first two right (14) and left (15) lamellae has substantially the shape of an elongated leaf.

4. Device according to claim 1 or 3, **characterized in that** that the right (14c) and left (15c) space delimited by the two first right (14) and left (15) lamellae is open above and below.

5. Device according to one of claims 1 to 4, **characterized in that** the first lamellae (31) and (32), (35) and (36), which medially delimit the elongated leaf-shaped space (31 c) and (32c), (35c) and (36c) respectively, are inserted in the middle portion (31 b) and (32b), (35b) and (36b) of the internal wall of the respective horizontal branch (13a) and (13b).

6. Device according to one of claims 1 to 5, **characterized in that** each elongated leaf-shaped space (29c) and (30c), (35c) and (36c) is closed at the top by a first lamella to form a pocket open at the bottom.

7. Device according to one of claims 1 to 6, **characterized in that** the two first lamellae (57) and (58), (61) and (62) have a wavy surface.

8. Device according to one of claims 1 to 7, **characterized in that** the first two lamellae (59) and (60), (63) and (64) are constituted by, or however they comprise, a spongy material.

9. Device according to one of claims 1 to 8, **characterized in that** in the proximal end of the internal wall of each of the two horizontal branches (13a) and (13b) there is at least one first vertical half tube-shaped lamella (37) and (38) which delimits an empty space (37a) and (38a).

10. Device according to one of claims 1 to 9, **characterized in that** the height measure of the first horizontal branches (13a) and (13b) is greater than the height measure of the connecting portion (13) that combines the first two horizontal branches (13a) and (13b).

11. Device according to one of claims 1 to 10, **characterized in that** each of the first two horizontal branches (13a) and (13b) is provided with an extension, in the lower part, in the shape of a half leaf with convexity toward the bottom, (65a) and (65b) arranged to narrow the columella.

12. Device according to one of claims 1 to 11, **characterized in that** each of the first two horizontal branches (13a) and (13b) is provided with an extension, in the lower part, in the shape of a half leaf (65a) and (65b) which makes a convexity arranged to be turned toward the floor of the vestibule and of the nasal cavity, when in use within the nose, in order to narrow the columella.

13. Device according to one or more of the claims 1-12, **characterized in that** the first two horizontal branches (13a), (13b) have two protrusions or third right (66) and left (67) lamellae, in the distal portion, suitable for being pushed manually towards each other in order to enlarge the first horizontal branches (13a) and (13b) facilitating the insertion around the columella (1) and the septum (11).

14. Device according to one or more of the claims 1-13, **characterized in that** it comprises, or it is constituted by, PVC, silicone or other soft and flexible material.

15. Device according to one of claims 1 to 14, **characterized in that** the first two horizontal branches (13a), (13b), are divergent from each other in undeformed configuration and they are suitable for being pushed manually in order to move the first horizontal branches (13a) and (13b) towards each other, facilitating the insertion around the columella (1) and the septum (11).

16. Device according to one of claims 1 to 15, **characterized in that** the second two dragonfly wing shaped lamellae suitable for being inserted into the nasal vestibule and the nasal valve, to the right (18) and to the left (19), have a length that varies from 4 to 18 mm.

17. Device according to one of claims 1 to 16, **characterized in that** the second two dragonfly wing shaped lamellae, suitable for being inserted into the nasal vestibule and the nasal valve, to the right (18) and to the left (19), have a length that varies from 4 to 15 mm.

18. Device according to one of claims 1 to 17, **characterized in that** the second two dragonfly wing shaped lamellae suitable for being inserted into the nasal vestibule and the nasal valve, to the right (18) and to the left (19), lie in a first ideal surface having a first inclination (α), with respect to the second branches (16b, 17b), preferably between 30 and 60°.

19. Device according to one of claims 1 to 18, **characterized in that** the second two dragonfly wing shaped lamellae, suitable for being inserted into the nasal vestibule and the nasal valve, to the right (18) and to the left (19), lie in a second surface having a second inclination (γ), with respect to the second branches (16b, 17b), preferably between 30 and 60°.

## Patentansprüche

1. Vorrichtung zum Beschränken des Nasenstegs (1) und zum Erweitern der Nasenflügel (3c, 2c) und der Nasenklappe (12g, 12f), mit mindestens zwei ersten horizontalen Zweigen (13a, 13b), die am distalen Ende (13) vereint sind, um ein U zu bilden; wobei von der ersten Wand der proximalen Enden (14a, 15a) erste zwei Lamellen - rechts (14) und links (15) - abzweigen, um so die interne Wand der jeweiligen horizontalen Zweige (13a, 13b) zu vereinen, wobei medial ein Raum nach rechts (14c) bzw. nach links (15c) abgegrenzt wird, wobei von der oberen Kante des proximalen Teils jedes horizontalen Zweigs (13a, 13b) nach rechts und nach links ein entsprechender zweiter Zweig (16b, 17b) abgeht; die letzteren spitzwinkeligen Kurven (16, 17), nach einem kurzen vertikalen Teil und hin zur Außenseite, setzen sich fort mit einer entsprechenden zweiten libellenflügelartigen Lamelle (18, 19) nach rechts und links, die sich zum Einführen in den Naseneingangsbereich und die Nasenklappe eignet.

2. Vorrichtung nach Anspruch 1, **dadurch gekennzeichnet, dass** die zweiten libellenflügelartigen Lamellen nach rechts (18) und nach links (19) sich zum Einführen in den Eingangsbereich und die Nasenklappe eignen, um so die Nasenflügel zu erweitern, wobei sie lateral in Bezug auf die Nasenscheidewand gedrückt werden.

3. Vorrichtung nach Anspruch 1, **dadurch gekennzeichnet dass** der rechte (14c) und linke (15c) Raum, der durch die ersten zwei rechten (14) und linken (15) Lamellen abgegrenzt wird, im Wesentlichen die Form eines länglichen Blattes hat.

4. Vorrichtung nach Anspruch 1 oder 3, **dadurch gekennzeichnet, dass** der rechte (14c) und linke (15c) Raum, der durch die zwei ersten rechten(14) und linken (15) Lamellen begrenzt wird, nach oben und unten offen ist.

5. Vorrichtung nach einem der Ansprüche 1 bis 4, **dadurch gekennzeichnet, dass** die ersten Lamellen (31) und (32), (35) und (36), die medial den länglichen blattförmigen Raum (31c) und (32c), (35c) bzw. (36c) begrenzen, in den mittleren Teil (31b) und (32b), (35b) und (36b) der internen Wand des jeweiligen horizontalen Zweigs (13a) und (13b) eingeführt werden.

6. Vorrichtung nach einem der Ansprüche 1 bis 5, **dadurch gekennzeichnet, dass** jeder längliche blattförmige Raum (29c) und (30c), (35c) und (36c) oben durch eine erste Lamelle geschlossen ist, um eine Tasche zu bilden, die am Boden offen ist.

7. Vorrichtung nach einem der Ansprüche 1 bis 6, **dadurch gekennzeichnet, dass** die ersten zwei Lamellen (57) und (58), (61) und (62) eine wellige Oberfläche haben.

8. Vorrichtung nach einem der Ansprüche 1 bis 7, **dadurch gekennzeichnet, dass** die ersten zwei Lamellen (59) und (60), (63) und (64) durch ein schwammiges Material gebildet werden oder ein solches umfassen.

9. Vorrichtung nach einem der Ansprüche 1 bis 8, **dadurch gekennzeichnet, dass** im proximalen Ende der internen Wand von jedem der zwei horizontalen Zweige (13a) und (13b) es mindestens eine erste vertikale halbe rohrförmige Lamelle (37) und (38) gibt, die einen leeren Raum (37a) und (38a) abgrenzt.

10. Vorrichtung nach einem der Ansprüche 1 bis 9, **dadurch gekennzeichnet, dass** das Höhenmaß der ersten horizontalen Zweige (13a) und (13b) größer als das Höhenmaß des Verbindungsteils (13) ist, der die ersten zwei horizontalen Zweige (13a) und (13b) kombiniert.

11. Vorrichtung nach einem der Ansprüche 1 bis 10, **dadurch gekennzeichnet, dass** jeder der ersten zwei horizontalen Zweige (13a) und (13b) mit einer Erweiterung versehen ist, im unteren Teil, in Form eines halben Blattes mit Konvexität zum Boden, (65a) und (65b), die zum Verengen des Nasenstegs ausgelegt ist.

12. Vorrichtung nach einem der Ansprüche 1 bis 11, **dadurch gekennzeichnet, dass** jeder der ersten zwei horizontalen Zweige (13a) und (13b) mit einer Erweiterung versehen ist, im unteren Teil, in Form eines halben Blattes (65a) und (65b), die bewirkt, dass eine Konvexität zum Boden des Nasen Eingangsbereichs und der Nasenhöhle angeordnet ist, wenn sie innerhalb der Nase in Gebrauch ist, um den Nasensteg zu verschmälern.

13. Vorrichtung nach einem oder mehreren der Ansprüche 1 bis 12, **dadurch gekennzeichnet, dass** die ersten zwei horizontalen Zweige (13a), (13b) zwei Vorwölbungen oder dritte rechte (66) und linke (67) Lamellen haben, in distalen Teil, dafür geeignet, von Hand aufeinander zu gestoßen zu werden, um die ersten horizontalen Zweige (13a) und (13b) zu vergrößern, was das Einführen rund um den Nasensteg (1) und die Trennwand (11) erleichtert.

14. Vorrichtung nach einem oder mehreren der Ansprüche 1 bis 13, **dadurch gekennzeichnet, dass** sie PVC, Silikon oder anderes weiches und flexibles Material umfasst oder aus demselben besteht.

15. Vorrichtung nach einem der Ansprüche 1 bis 14, **dadurch gekennzeichnet, dass** die ersten zwei horizontalen Zweige (13a), (13b) voneinander in der nicht deformierten Konfiguration abweichen und sie sich dafür eignen, manuell gedrückt zu werden, um die ersten horizontalen Zweige (13a) und (13b) zueinander zu bewegen, was das Einführen rund um den Nasensteg (1) und die Trennwand (11) erleichtert.

16. Vorrichtung nach einem der Ansprüche 1 bis 15, **dadurch gekennzeichnet, dass** die zweiten zwei libellenflügelartigen Lamellen, die sich dazu eignen, in den Naseneingangsbereich und die Nasenklappe eingeführt zu werden, nach rechts (18) und nach links (19), eine Länge haben, die zwischen 4 und 18 mm variiert.

17. Vorrichtung nach einem der Ansprüche 1 bis 16, **dadurch gekennzeichnet, dass** die zweiten zwei libellenflügelartigen Lamellen, die sich dazu eignen, in den Naseneingangsbereich und die Nasenklappe eingeführt zu werden, nach rechts (18) und nach links (19), eine Länge haben, die zwischen 4 und 15 mm variiert.

18. Vorrichtung nach einem der Ansprüche 1 bis 17, **dadurch gekennzeichnet, dass** die zweiten zwei libellenflügelartigen Lamellen, die sich dazu eignen, in den Naseneingangsbereich und die Nasenklappe eingeführt zu werden, nach rechts (18) und nach links (19), in einer ersten idealen Fläche liegen, die eine erste Neigung (α) gegenüber den zweiten Zweigen (16b, 17b) haben, vorzugsweise zwischen 30 und 60°.

19. Vorrichtung nach einem der Ansprüche 1 bis 18, **dadurch gekennzeichnet, dass** die zweiten zwei libellenflügelartigen Lamellen, die sich dazu eignen, in den Naseneingangsbereich und die Nasenklappe eingeführt zu werden, nach rechts (18) und nach links (19), in einer zweiten Fläche liegen, die eine zweiten Neigung (y) gegenüber den zweiten Zweigen (16b, 17b) hat, vorzugsweise zwischen 30 und 60°.

## Revendications

1. Dispositif pour rétrécir la columelle nasale (1) et élargir les ailes nasales (3c) et (2c) et la valve nasale (12g) et (12f), qui comprend au moins deux premières branches horizontales (13a) et (13b) réunies, au niveau de l'extrémité distale (13), pour former un U ; dans lequel, à partir de la paroi interne de l'extrémité proximale (14a) et (15a), deux premières lamelles - droite (14) et gauche (15) - bifurquent, de façon à rejoindre la paroi interne des branches horizontales respectives (13a, 13b) délimitant, en position médiale, un espace, à droite (14c) et à gauche (15c), respectivement, et à partir du bord supérieur de la partie proximale de chaque branche horizontale (13a) et (13b), à droite et à gauche, une seconde branche respective (16b, 17b) se détache ; cette dernière forme des coudes à angle aigu (16, 17), après une courte partie verticale, et vers l'extérieur, et elle se prolonge avec une deuxième lamelle respective en forme d'aile de libellule (18, 19), à droite et à gauche, appropriée pour être introduite dans le vestibule nasal et la valve nasale.

2. Dispositif selon la revendication 1, **caractérisé par le fait que** les deuxièmes lamelles en forme d'aile de libellule, à droite (18) et à gauche (19), sont appropriées pour être introduites dans le vestibule et la valve nasale de façon à élargir les ailes nasales, en les poussant latéralement par rapport à la cloison nasale.

3. Dispositif selon la revendication 1, **caractérisé par le fait que** l'espace droit (14c) et gauche (15c) délimité par les deux premières lamelles droite (14) et gauche (15) a sensiblement la forme d'une feuille allongée.

4. Dispositif selon la revendication 1 ou 3, **caractérisé par le fait que** l'espace droit (14c) et gauche (15c) délimité par les deux premières lamelles droite (14) et gauche (15) est ouvert au-dessus et au-dessous.

5. Dispositif selon l'une des revendications 1 à 4, **caractérisé par le fait que** les premières lamelles (31) et (32), (35) et (36), qui délimitent en position médiale l'espace en forme de feuille allongée (31c) et (32c), (35c) et (36c) respectivement, sont introduites dans la partie centrale (31b) et (32b), (35b) et (36b) de la paroi interne de la branche horizontale respective (13a) et (13b).

6. Dispositif selon l'une des revendications 1 à 5, **caractérisé par le fait que** chaque espace en forme de feuille allongée (29c) et (30c), (35c) et (36c) est fermé à la partie supérieure par une première lamelle pour former une poche ouverte à la partie inférieure.

7. Dispositif selon l'une des revendications 1 à 6, **caractérisé par le fait que** les deux premières lamelles (57) et (58), (61) et (62) ont une surface ondulée.

8. Dispositif selon l'une des revendications 1 à 7, **caractérisé par le fait que** les deux premières lamelles (59) et (60), (63) et (64) sont constituées par, ou autrement comprennent, une matière spongieuse.

9. Dispositif selon l'une des revendications 1 à 8, **caractérisé par le fait que**, dans l'extrémité proximale de la paroi interne de chacune des deux branches horizontales (13a) et (13b), il y a au moins une première lamelle verticale en forme de demi-tube (37) et (38) qui délimite un espace vide (37a) et (38a).

10. Dispositif selon l'une des revendications 1 à 9, **caractérisé par le fait que** la mesure de hauteur des premières branches horizontales (13a) et (13b) est plus grande que la mesure de hauteur de la partie de liaison (13) qui combine les deux premières branches horizontales (13a) et (13b).

11. Dispositif selon l'une des revendications 1 à 10, **caractérisé par le fait que** chacune des deux premières branches horizontales (13a) et (13b) comporte une extension, dans la partie inférieure, sous la forme d'une demi-feuille ayant une convexité dirigée vers la partie inférieure, (65a) et (65b) agencée pour rétrécir la columelle.

12. Dispositif selon l'une des revendications 1 à 11, **caractérisé par le fait que** chacune des deux premières branches horizontales (13a) et (13b) comporte une extension, dans la partie inférieure, sous la forme d'une demi-feuille (65a) et (65b) qui forme une convexité agencée pour être tournée vers le plancher du vestibule et de la cavité nasale, lors de l'utilisation dans le nez, afin de rétrécir la columelle.

13. Dispositif selon une ou plusieurs des revendications 1 à 12, **caractérisé par le fait que** les deux premières branches horizontales (13a), (13b) ont deux saillies ou des troisièmes lamelles droite (66) et gauche (67), dans la partie distale, appropriées pour être poussées manuellement l'une vers l'autre de façon à agrandir les premières branches horizontales (13a) et (13b), facilitant l'introduction autour de la columelle (1) et la cloison (11).

14. Dispositif selon une ou plusieurs des revendications 1 à 13, **caractérisé par le fait qu'**il comprend ou est constitué de PVC, de silicone ou autre matériau souple et flexible.

15. Dispositif selon l'une des revendications 1 à 14, **caractérisé par le fait que** les deux premières branches horizontales (13a), (13b) divergent l'une de l'autre dans une configuration non déformée et sont appropriées pour être poussées manuellement afin de rapprocher les premières branches horizontales (13a) et (13b) l'une vers l'autre, facilitant l'introduction autour de la columelle (1) et la cloison (11).

16. Dispositif selon l'une des revendications 1 à 15, **caractérisé par le fait que** les deux deuxièmes lamelles en forme d'aile de libellule appropriées pour être introduites dans le vestibule nasal et la valve nasale, à la droite (18) et à la gauche (19), ont une longueur qui varie de 4 à 18 mm.

17. Dispositif selon l'une des revendications 1 à 16, **caractérisé par le fait que** les deux deuxièmes lamelles en forme d'aile de libellule, appropriées pour être introduites dans le vestibule nasal et la valve nasale, à la droite (18) et à la gauche (19), ont une longueur qui varie de 4 à 15 mm.

18. Dispositif selon l'une des revendications 1 à 17, **caractérisé par le fait que** les deux deuxièmes lamelles en forme d'aile de libellule appropriées pour être introduites dans le vestibule nasal et la valve nasale, à la droite (18) et à la gauche (19), s'étendent dans une première surface idéale ayant une première inclinaison (α), par rapport aux secondes branches (16b, 17b), de préférence entre 30 et 60°.

19. Dispositif selon l'une des revendications 1 à 18, **caractérisé par le fait que** les deux deuxièmes lamelles en forme d'aile de libellule, appropriées pour être introduites dans le vestibule nasal et la valve nasale, à la droite (18) et à la gauche (19), s'étendent dans une seconde surface ayant une seconde inclinaison (y), par rapport aux secondes branches (16b, 17b), de préférence entre 30 et 60°.
